# EUROPÄISCHE PATENTSCHRIFT

(11) **EP 1 837 039 B1**
(45) Veröffentlichungstag und Bekanntmachung des Hinweises auf die Patenterteilung: **17.08.2016**
(21) Anmeldenummer: 07011684.3
(22) Anmeldetag: 08.05.2001
(51) Int. Cl.: A61L 24/10

(54) **Verwendung von fibrinogenhaltigem Gewebekleber zur Verhinderung von Gewebsadhäsionen**
Cloth adhesive with improved anti-adhesive properties
Adhésif tissulaire doté de propriétés anti-adhésives améliorées

(30) Priorität: 22.05.2000 DE 10025001
(43) Veröffentlichungstag der Anmeldung: 26.09.2007
(62) Teilanmeldung aus: 01111013.7
(73) Patentinhaber: CSL Behring GmbH, 35041 Marburg (DE)
(72) Erfinder: Metzner, Hubert, Dr., 35041 Marburg (DE); Dickneite, Gerhard, Prof., 35043 Marburg (DE); Kröz, Monika, Dr., 88499 Altheim-Waldhausen (DE)

(56) Entgegenhaltungen:
- WO-A-99/29338
- DE IACO PIERANDREA ET AL.: "Fibrin sealant in laparoscopic adhesion prevention in the rabbit uterine horn model", FERTILITY AND STERILITY, Bd. 62, Nr. 2, 1. August 1994 (1994-08-01) , Seiten 400-404, XP001025656,
- LINDENBERG S. ET AL.: "Prevention of peritoneal adhesion formation by fibrin sealant", ANNALES CHIRURGIAE ET GYNAECOLOGIAE, Bd. 73, Nr. 1, 1. Januar 1984 (1984-01-01) , Seiten 11-13, XP001025655,
- BUNCE L.A. ET AL.: "Endothelial cell spreading on fibrin requires fibrinopeptide B cleavage and amino acid residues 15-42 on the beta-chain", J. CLIN. INVEST., vol. 89, no. 3, 1992, pages 842-850,

## Beschreibung

Gegenstand der Erfindung ist die Verwendung eines Gewebeklebers zur Verminderung oder Verhinderung von postoperativen Gewebsadhäsionen, der sich gegenüber bekannten Gewebeklebern durch verbesserte antiadhäsive Eigenschaften auszeichnet.

Es ist bekannt, daß bei der Entwicklung von Gewebeklebern bisher die hämostatische Wirksamkeit oder die Abdichtung (z.B. gegen Liquorverlust) im Vordergrund stand. Diese Indikationen machen auch heute noch die ganz wesentliche Anzahl der Anwendungen von Gewebeklebern aus.

Allerdings ist in der Vergangenheit auch - mit unterschiedlichem Erfolg - die präklinische oder klinische Anwendung von Gewebeklebern zur Vermeidung von Adhäsionen nach operativen Eingriffen beschrieben worden. So berichteten H. Moro et al. von einer Inhibierung perikardialer Adhäsionen in einem Modell am Hund (H. Moro, J. Hayashi, H. Ohzeki, T. Nakayama, O. Namura, K. Hanzawa und N. Yagi. Jap J Thor Cardiovasc Surg 47: 79-84,1999). Auch H. Takeuchi et al. und P.A. De laco et al. beschrieben den erfolgreichen Einsatz von Gewebeklebern zur Vermeidung bzw. Reduktion von Adhäsionen am Uterushorn des Kaninchens (H. Takeuchi, Y. Toyonari, N. Mitsuhashi und Y. Kuwabara. J Obstet Gynaecol 23: 479-484, 1997; P.A. De laco, A. Costa, G. Mazzoleni, G. Pasquinelli, L. Bassein und A. Marabini. Fertil Steril 62:400-404, 1994). Ebenfalls von S. Lindenberg et al. wurde die Verringerung peritonealer Adhäsionen durch den Einsatz von Gewebeklebern in einem Modell an der Ratte beschrieben (S. Lindenberg und J.G. Lauritsen. Annales Chirurgiae et Gynecologiae 73: 11-13, 1984). Allerdings gab es auch andere Autoren, die beim Einsatz von Fibrinklebern gegenüber einer unbehandelten Kontrolle keine Verringerung von Adhäsionen beobachten konnten. Dies waren unter anderem J.F.H. Gauwerki, J. Mann und G. Bastert, Arch Gynäkol Obstet 247:161 (1990) und V.A.C. Evrard, A. De Bellis, W. Boeckx und I.A. Brosens, Hum Reprodt 11:1877-1880 (1996). Die sich teilweise widersprechenden Berichte dürften im Wesentlichen darauf zurückzuführen sein, daß der durch die vorhandenen Produkte erzielbare Effekt nicht ausreichend groß bzw. deutlich ist, um durchgehend zu eindeutigen Resultaten zu führen.

In jüngster Zeit wurde die Verwendbarkeit von Fibrinschichten zur Vermeidung von Adhäsionen auch in der Patentliteratur erwähnt. Die internationale Patentanmeldung WO 96/22115 beschreibt ein folienartiges Material, das aus quervernetztem Fibrin besteht und das zur Verhinderung von Adhäsionen eingesetzt wird, selbst jedoch keine hämostatischen Eigenschaften besitzt. In einer anderen Ausführungsform wird dieses Material in situ erzeugt, in dem es nach einem ersten hämostatisch wirksamen Gewebekleber als zweite Gewebekleberschicht ohne hämostatische Eigenschaften angewendet wird. Diese Verfahren sind jedoch entweder unpraktikabel, da die Fixierung eines solchen Fibrinfilms schwierig ist oder umständlich, da zwei Gewebekleber eingesetzt werden müssen, um sowohl hämostatische Wirksamkeit als auch anti-adhäsive Eigenschaften zu erzielen.

Außerdem ist auch eine Zubereitung aus Fibrin oder Fibrinogen und einem bioverträglichen oder bioabbaubaren, eine viskose Lösung bildenden Polymeren mit anti-adhäsiven Eigenschaften aus der internationalen Patentanmeldung WO 92/22312 bekannt.

Es stellte sich deshalb die Aufgabe, einen Gewebekleber zu entwickeln, der bei guten hämostatischen Eigenschaften verbesserte Ergebnisse bei der Verminderung oder Verhinderung von Gewebsadhäsionen zeigt und der darüber hinaus ohne den Zusatz viskose Lösungen bildender Polymeren mit antiadhäsiven Eigenschaften auskommt.

Wegen ihrer großen medizinischen Bedeutung sind in den letzten Jahren erhebliche Forschungsanstrengungen unternommen worden, um die bekannten Gewebekleber weiter zu entwickeln und zu verbessern. Besonderes Augenmerk wurde dabei auch auf die Verbesserung der Lagerfähigkeit von Gewebeklebern gelegt. So sind in den deutschen Patentanmeldungen DE-A-198 53 033, DE-A-198 61 158 und DE-A 100 12 732 Gewebekleber bzw. deren Komponenten beschrieben, die sich u.a. durch eine besonders hohe Lagerfähigkeit im flüssigen und/oder gefrorenen Zustand auszeichnen. Die genauere Untersuchung dieser neuen Gewebekleber hat nun gezeigt, daß sie noch weitere vorteilhafte Eigenschaften aufweisen, die ihnen zusätzliche und wertvolle Anwendungsmöglichkeiten erschließen.

Es hat sich nämlich erwiesen, daß diese neuen Gewebekleber über erheblich verbesserte anti-adhäsive Eigenschaften verfügen, ohne daß dabei Einbußen in ihren hämostatischen Eigenschaften hingenommen werden müssen. Die besonderen, anti-adhäsiven Eigenschaften der neuen Gewebekleber zeigen sich sowohl gegenüber unbehandelten als auch gegenüber mit herkömmlichen Gewebeklebern behandelten Wunden. Überraschend ist dabei insbesondere, daß auch beim Vergleich mit herkömm-lichen Gewebeklebern deutlich verbesserte Effekte bei der Verminderung oder Verhinderung von Gewebsadhäsionen erzielt werden, wenn die vorstehend genannten neuen Gewebekleber eingesetzt werden. Diese Effekte wurden sowohl in einem typischen Tiermodell zur Untersuchung der Adhäsionsverminderung wie einer Längsschnittwunde am Uterushorn des Kaninchens, als auch bei der hämostatischen Anwendung einer partiellen Leberresektion am Kaninchen beobachtet.

Gegenstand der Erfindung ist deshalb die Verwendung eines Gewebeklebers enthaltend
- eine stabilisierte, im flüssigen und/oder eingefrorenen Zustand lagerfähige Fibrinogenzubereitung, der eine chaotrope Substanz zugesetzt ist, und
- eine Thrombinzubereitung
zur Verminderung oder Verhinderung von postoperativen Gewebsadhäsionen.

Dabei kann dem Gewebekleber zusätzlich eine den Blutgerinnungsfaktor XIII enthaltende Zubereitung zugesetzt werden, sofern dieser nicht in ausreichender Menge enthalten ist, so daß er als 3-Komponenten-Kleber verwendet wird. Denn eine möglichst vollständige Fibrinquervernetzung kann den antiadhäsiven Effekt eines Fibrinklebers verstärken indem die Fibrinmatrix z.B. einem fibrinolytischen Abbau weniger zugänglich ist. Es ist allerdings auch möglich, den Blutgerinnungsfaktor XIII von Anfang an der Fibrinogenzubereitung zuzumischen, so daß ein 2-Komponenten-Kleber zum Einsatz kommt. Im Falle eines 3-Komponentenklebers kann das Mischungsverhältnis der Komponenten Fibrinogen, Faktor XIII und Thrombin in geeigneter Weise gewählt werden, um gute mechanische Eigenschaften des Klebers zu erzielen. Geeignet sind z.B. Mischungsverhältnisse von ca. 1:1:1 bis ca. 10:1:1 oder 10:1:2 oder generell x:y:z wobei x ≥ z ≥ y.

Der erfindungsgemäß verwendete Gewebekleber enthält in der Fibrinogenzubereitung eine chaotrope Substanz. Als geeignete chaotrope Substanzen haben sich z.B. Arginin, Guanidin, Citrullin, Harnstoff oder deren Derivate oder ihre Mischungen erwiesen. Sie werden der Fibrinogenzubereitung im allgemeinen in Mengen von 0,1 bis 1,0 Mol/l, vorzugsweise in Mengen von unter 0,5 Mol/l, beigegeben.

Die Eigenschaften der vorstehend genannten neuen Gewebekleberwerden weiterhin durch den Zusatz eines Antifibrinolytikums vorteilhaft beeinflusst. Als Antifibrinolytikum werden z.B. Aprotinin, ε-Aminocapronsäure (EACA), p-Aminomethylbenzoesäure (PAMBA) oder eines ihrer physiologisch verträglichen Salze oder Derivate verwendet.

Außerdem können in der Fibrinogenzubereitung als Stabilisatoren
- ein anorganisches Salz oder
- ein oder mehrere physiologisch verträgliche Salze von organischen Carbonsäuren, insbesondere der Zitronensäure oder der Milchsäure, oder
- eine oder mehrere Aminosäuren oder
- ein Mono- oder Disaccharid oder
- ein Zuckeralkohol
oder eine ihrer Mischungen enthalten sein.

Einen positiven Effekt auf die antiadhäsiven Eigenschaften der beanspruchten, verbesserten Fibrinkleber kann man weiterhin durch geeignete Reinigungsmethoden erzielen, z.B. indem man den Plasminogengehalt der Fibrinogenkomponente verringert. Solche Methoden können z.B. die Immunaffinitätschromatographie über gekoppelte Antikörper oder die Affinitätschromatographie über Aminogruppen-haltige Träger sein. Deshalb umfasst diese Erfindung u.a. auch Fibrinkleber mit Fibrinogenkomponenten, deren Plasminogengehalte signifikant reduziert sind. Solche Fibrinogenkomponenten weisen bevorzugt ein Verhältnis von Plasminogen zu Fibrinogen von < 1.8 x 10⁻⁴ (w/w) auf, besonders bevorzugt von < 10⁻⁴ (w/w).

Die dem erfindungsgemäß einzusetzenden Gewebekleber hinzugefügte Faktor XIII-Zubereitung muss ebenfalls stabilisiert werden, wenn sie nicht dem bereits stabilisierten Fibrinogen zugegeben wird. In diesem Fall ist es vorteilhaft, der Faktor XIII-Zubereitung ein physiologisch verträgliches Salz einer organischen Di-, Tri- oder Tetracarbonsäure, insbesondere der Zitronensäure, und gegebenenfalls weitere Stabilisatoren und/oder Puffersubstanzen für den Faktor XIII zuzugeben. Als weitere Stabilisatoren kommen dabei
- ein Mono- oder Disaccharid oder ein Zuckeralkohol und/oder
- eine Aminosäure aus der Gruppe Glycin, Glycylglycin, Alanin, Cystein, Histidin, Glutamin oder ein physiologisch verträgliches Salz der Glutamin- oder Asparagin-säure und/oder
- ein reduzierendes oder oxidationsverhinderndes Agens und/oder
- eine oberflächenaktive Substanz in Betracht.

Sie werden üblicherweise in einer Menge von bis zu 5 Gew.% der Faktor XIII-Zubereitung zugesetzt. Gewebekleber dieser Art sind in den deutschen Patentanmeldungen DE-A-198 53 033 und DE-A-198 61 158 beschrieben.

Die in dem erfindungsgemäß eingesetzten Gewebekleber enthaltene Thrombinzubereitung weist in einer Ausführungsform als besonderes Merkmal auf, daß sie als Stabilisator einen nicht-kovalent bindenden Inhibitor enthalten kann. Geeignete Substanzen hierfür sind Verbindungen wie Benzamidin oder p-Aminobenzamidin und andere niedrig- bis mittelaffine Protease-Inhibitoren. Durch den Zusatz dieser niedrig- oder mittelaffinen Inhibitoren wird die Aktivität von Thrombin gegenüber Substanzen wie Fibrinogen nicht wesentlich beeinträchtigt. Der Thrombinzubereitung können außerdem neben einem löslichen Calciumsalz zur Stabilisierung Natriumchlorid, ein Zucker oder ein Zuckeralkohol und/oder eine Aminosäure oder auch das Salz einer Mono- oder Polycarbonsäure und/oder das Salz einer Mono- oder Polyhydroxycarbonsäure oder Mischungen der genannten Stabilisatoren zugesetzt werden.

Das hierfür verwendete Thrombin wird aus dem aus Plasma oder aus einer Plasmafraktion gewonnenen Prothrombin hergestellt. Nach dessen Aktivierung zu Thrombin ohne Zusatz von Thromboplastin sowie ggf. weiteren Aufarbeitungsschritten kann es durch eine hydrophobe Interaktionschromatographie und/oder eine Kationenaustauschchromatographie gereinigt werden. Dieses Verfahren ist im einzelnen in der deutschen Patentanmeldung DE-A-100 12 732 beschrieben. Besonders vorteilhaft ist es dabei, wenn der Gewebekleber oder seine Bestandteile auch noch einem oder mehreren Verfahren zur Inaktivierung von Viren unterworfen werden.

Als Ausgangsmaterial für die Herstellung der einzelnen Komponenten der erfindungsgemäßen Fibrinkleber können außer Plasmafraktionen auch rekombinante Proteine, hergestellt durch Isolierung aus Zellkulturen oder Zellkulturüberständen, verwendet werden.

Zur Untersuchung der Auswirkungen dieser verbesserten Gewebekleber auf die Verhinderung oder Verminderung von postoperativen Gewebsadhäsionen wurde als Beispiel ein verbesserter Gewebekleber folgender Zusammensetzung hergestellt:
Fibrinogenkomponente enthaltend:
   90 mg/ml Fibrinogen-Konzentrat,
   100 mMol/l NaCl,
   20 mMol/l Na₃-Citrat x 2 H₂O,
   237 mMol/l L-Arginin x HCl und
   80 mMol/l ε-Aminocapronsäure oder 1000 KIE/ml Aprotinin
Faktor XIII-Komponente enthaltend:
   120 E/ml Faktor XIII-Konzentrat,
   10 mMol/l Na₃-Citrat x 2 H₂O,
   50 m/Mol/l L-Histidin
Thrombin-Komponente enthaltend:
   1500 IE/ml Thrombin-Konzentrat,
   150 mMol/l NaCl,
   40 mMol/l CaCl₂,
   110 mMol/l Mannitol,
   5 mMol/l L-Histidin.

Der pH-Wert nach Mischung der Komponenten zum Gewebekleber betrug ca. 7,4.

Die Verwendung dieses Gewebeklebers bei Operationen wird im Folgenden beispielhaft beschrieben:

### Beispiel 1: Verhinderung von Adhäsionen am Uterushorn.

An 12 weiblichen Kaninchen wurden unter Narkose nach Eröffnung der Bauchhöhle longitudinale Inzisionen an den Uterushörnern gesetzt. Die Inzisionen wurden mit chirurgischem Nahtmaterial wieder verschlossen. Jeweils sechs Kaninchen wurden folgenden zwei Behandlungsgruppen zugeteilt: 1. Keine Behandlung, 2. Behandlung mit verbessertem Gewebekleber. Die Wunden der 2. Gruppe wurden jeweils vollständig mit Gewebekleber bedeckt. Nach Schließen der Bauchhöhle konnten die Tiere wieder aufwachen. Nach sieben Tagen wurden die Kaninchen euthanasiert und die Adhäsionen der Uteri mit dem umgebenden Gewebe beurteilt. Ausgeschlossen von der Auswertung wurden Verwachsungen beider Inzisionen miteinander. Die Untersuchungsergebnisse sind in der Tabelle 1 dargestellt.

Die unbehandelten Tiere zeigten in 63,6% aller Fälle Adhäsionen. Eine deutliche Reduktion der Adhäsionen wurde in der mit verbessertem Gewebekleber behandelten Gruppe beobachtet. Hier lag die Frequenz der Adhäsionen lediglich bei 11,1%.

**Tabelle 1: Uterus-Adhäsionen mit dem umliegenden Gewebe nach der Behandlung mit Gewebeklebern**

| | **1. Keine Behandlung** | **2. Verbesserter Gewebekleber** |
|---|---|---|
| Frequenz der Adhäsionen | 63,6% | 11.1% |

### Beispiel 2: Verhinderung von Adhäsionen am Uterushorn

In diesem Experiment wurde der verbesserte Gewebekleber mit einem kommerziellen Kleber (Beriplast® P) und keiner Behandlung an insgesamt 36 Kaninchen verglichen. Es wurden entsprechend der im Beispiel 1 beschriebenen Methode drei Gruppen mit jeweils 12 Tieren gebildet, bei denen pro Tier ein Uterushorn wie folgt behandelt wurde: 1. keine Behandlung, 2. Beriplast^{®} P, 3. Verbesserter Gewebekleber. Die Frequenz und die Ausdehnung der Adhäsionen wurden am Tag 7 beurteilt. Die Ergebnisse sind in der Tabelle 2 zusammengefasst.

Alle Tiere in der Gruppe, die keine Behandlung mit einem Gewebekleber erhielten, zeigten Adhäsionen (100%). Die mit Beriplast® P behandelten Kaninchen hatten eine deutlich geringere Frequenz von Adhäsionen (75%). Die geringste Adhäsionsfrequenz wurde in der Gruppe von Tieren, die mit verbessertem Gewebekleber behandelt wurden, beobachtet. Die Ausdehnung der Adhäsionen (Länge in cm) ergab ähnliche Befunde.

**Tabelle 2: Uterus-Adhäsion mit dem umliegenden Gewebe nach der Behandlung mit Fibrinklebern**

| | **1. Keine Behandlung** | **2. Beriplast^{®} P** | **3. Verbesserter Gewebekleber** |
|---|---|---|---|
| Frequenz der Adhäsion (%) | 100% | 75% | 50% |
| Länge der Adhäsionen (cm) | 1,52 | 1,03 | 0,67 |

### Beispiel 3: Verhinderung von Adhäsionen am Uterushorn.

In einem weiteren Experiment wurden verbesserte Gewebekleber mit einem kommerziellen Kleber (Beriplast^{®} P) sowie einer unbehandelten Kontrolle verglichen. Es wurden entsprechend der in Beispiel 2 beschriebenen Methode mehrerer Gruppen mit jeweils 12 Tieren gebildet, wobei pro Tier nur ein Uterushorn verwendet wurde. Die Tiere wurden wie folgt behandelt:
1. Keine Behandlung
2. Beriplast^{®} P
3. Verbesserter Gewebekleber
4. Verbesserter Gewebekleber (Aprotinin anstelle EACA)
5. Verbesserter Gewebekleber mit reduziertem Plasminogengehalt

Die Frequenz und die Ausdehnung der Adhäsionen wurden am Tag 7 beurteilt. Tabelle 3 zeigt die Ergebnisse der Studie.

Ca. zwei Drittel der Tiere, die keine Behandlung mit einem Gewebekleber erhielten, zeigten Adhäsionen (66,7%). Eine deutlich geringere Frequenz von Adhäsionen bzw. die geringste Adhäsionsfrequenz wurde in der Gruppe von Tieren beobachtet, die mit verbesserten Gewebeklebern behandelt wurden. Die Ausdehnung der Adhäsionen (Länge in cm) ergab ähnliche Befunde.

**Tabelle 3: Adhäsionen des Uterus mit dem umliegenden Gewebe nach der Behandlung mit unterschiedlichen Fibrinklebern (Mittelwerte für n=12 Tiere)**

| | **1. Unbehandelte Kontrolle** | **2. Beriplast^{®} P** | **3. Verbesserter Gewebekleber** | **4. Verbesserter Gewebekleber (Aprotinin anstelle EACA)** | **5. Verbesserter Gewebekleber mit reduziertem Plasminogen-Gehalt** |
|---|---|---|---|---|---|
| Frequenz der Adhäsion (%) | 66,7% | 41,7% | 33,3% | 16,7% | 0% |
| Länge der Adhäsionen (cm) | 0,59 | 0,19 | 0,26 | 0,13 | 0 |

### Beispiel 4: Verhinderung von Adhäsionen am Uterushorn.

In diesem Experiment wurden entsprechend der in Beispiel 1 beschriebenen Methode mehrerer Gruppen mit jeweils 8 Tieren gebildet, bei denen beide Uterushörner operiert wurden. Es wurden verbesserte Gewebekleber mit einer Kontrollgrupe ohne Behandlung an 16 Uterushörnern pro Gruppe eingesetzt. Folgende Behandlungsgruppen wurden verglichen:
1. Keine Behandlung
2. Verbesserter Gewebekleber mit reduziertem Plasminogengehalt
3. Verbesserter Gewebekleber mit zunächst reduziertem und vor Anwendung wieder aufgestocktem Plasminogengehalt

Es wurden nur solche Uterushörner ausgewertet, die nicht an der Inzision des anderen Uterushorns adhärierten. Die Ergebnisse dieser Versuchsreihe (siehe Tabelle 4) zeigen, dass die Abreicherung von Plasminogen die antiadhäsiven Eigenschaften eines Fibrinklebers weiter verbessern kann.

**Tabelle 4: Verhinderung von Adhäsionen am Uterushorn durch Behandlung mit Fibrinklebern (Mittelwerte)**

| | **1. Keine Behandlung** | **2. Verbesserter Gewebekleber mit reduziertem Plasminogen-Gehalt** | **3. Verbesserter Gewebekleber nach Reduktion des Plasminogen-Gehalts und Aufstockung mit Plasminogen** |
|---|---|---|---|
| Frequenz der Adhäsion (%) | 68.8% | 15.4% | 46.2% |
| Länge der Adhäsionen (cm) | 0,53 | 0,07 | 0,32 |

### Beispiel 5: Verhinderung von Adhäsionen nach Leberresektion

14 Kaninchen wurden anästhesiert und die Leber nach der Eröffnung der Bauchhöhle dargestellt. Von einem Leberlappen wurde ein Stück von ca. 3,5 g reseziert, wobei eine Wunde von ca. 4 cm² entstand. Die Wunde wurde zur Blutstillung vollständig mit einem Gewebekleber bedeckt, wobei jeweils sieben Kaninchen Beriplast^{®} P oder verbesserten Gewebekleber erhielten. Die Anzahl der Tiere mit kompletter Blutstillung wurde über fünf Minuten ermittelt. Danach wurde die Bauchhöhle wieder verschlossen und die Anästhesie beendet. Nach sieben Tagen wurden die Tiere euthanasiert und die Adhäsionen der Leber mit dem angrenzenden Gewebe beurteilt.

Tabelle 5 zeigt, daß die Anzahl der Adhäsionen in der Gruppe, die mit verbessertem Gewebekleber behandelt wurde, deutlich geringer war als in der Gruppe, die mit Beriplast® P behandelt worden war. Alle Tiere wiesen eine komplette Blutstillung auf.

**Tabelle 5: Haemostase und Adhäsionen der Leber mit dem umliegenden Gewebe nach der Behandlung mit Gewebeklebern**

| | **1. Beriplast^{®} P** | **2. Verbesserter Gewebekleber** |
|---|---|---|
| **Anzahl der Tiere mit Adhäsionen** | **5/7 (71.4%)** | **2/7 (28.6%)** |
| **Anzahl der Tiere mit kompletter Blutstillung** | **7/7 (100%)** | **7/7 (100%)** |

## Patentansprüche

1. Gewebekleber enthaltend
- eine stabilisierte, im flüssigen und/oder eingefrorenen Zustand lagerfähige Fibrinogen-Zubereitung, der eine chaotrope Substanz zugesetzt ist, und
- eine Thrombin Zubereitung
zur Verwendung bei der Verminderung oder Verhinderung von postoperativen Gewebsadhäsionen.

2. Gewebekleber nach Anspruch 1, **dadurch gekennzeichnet, dass** er zusätzlich eine den Blutgerinnungsfaktor XIII enthaltende Zubereitung umfasst, die auch mit der Fibrinogen-Zubereitung vermischt sein kann.

3. Gewebekleber nach den Ansprüchen 1 und 2, **dadurch gekennzeichnet, dass** er ein Antifibrinolytikum enthält.

4. Gewebekleber nach Anspruch 3, **dadurch gekennzeichnet, dass** er als Antifibrinolytikum die ε-Aminocapronsäure enthält.

5. Gewebekleber nach Anspruch 3, **dadurch gekennzeichnet, dass** er als Antifibrinolytikum die p-Aminomethylbenzoesäure enthält.

6. Gewebekleber nach Anspruch 3, **dadurch gekennzeichnet, dass** er als Antifibrinolytikum Aprotinin enthält.

7. Gewebekleber nach Anspruch 1-6, **dadurch gekennzeichnet, dass** der Plasminogengehalt der Fibrinogen-Komponente durch eine Immunaffinitätschromatographie über gekoppelte Antikörper oder eine Affinitätschromatographie über Aminogruppen-haltige Träger verringert wurde.

8. Gewebekleber nach Anspruch 1-7, **dadurch gekennzeichnet, dass** die Fibrinogen-Komponente einen reduzierten Plasminogengehalt aufweist, wobei das Verhältnis von Plasminogen zu Fibrinogen bei < 1,8 x 10⁻⁴ (w/w) liegt.

9. Gewebekleber nach den Ansprüchen 2 bis 8, **dadurch gekennzeichnet, dass** der den Faktor XIII enthaltenden Zubereitung
- ein physiologisch verträgliches Salz einer organischen Di-, Tri- oder Tetracarbonsäure, insbesondere der Zitronensäure, und
- gegebenenfalls weitere Stabilisatoren und/oder Puffersubstanzen für den Faktor XIII zugesetzt sind.

10. Gewebekleber nach Anspruch 9, **dadurch gekennzeichnet, dass** der den Faktor XIII enthaltenden Zubereitung als weitere Stabilisatoren
- ein Mono- oder Disaccarid oder ein Zuckeralkohol und/oder
- eine Aminosäure aus der Gruppe Glycin, Glycylglycin, Alanin, Cystein, Histidin, Glutamin oder ein physiologisch verträgliches Salz der Glutamin- oder Asparaginsäure und/oder
- ein reduzierendes oder oxydationsverhinderndes Agens und/oder
- eine oberflächenaktive Substanz zugesetzt sind.

11. Gewebekleber nach den Ansprüchen 1 bis 10, **dadurch gekennzeichnet, dass** der Fibrinogen-Zubereitung eine oder mehrere chaotrope Substanzen aus der Gruppe bestehend aus Arginin, Guanidin, Citrullin, Harnstoff oder deren Derivaten oder ihre Mischungen zugesetzt sind.

12. Gewebekleber nach den Ansprüchen 1 bis 11, **dadurch gekennzeichnet, dass** der Fibrinogen-Zubereitung zusätzlich als Stabilisatoren
- ein anorganisches Salz oder
- eine oder mehrere physiologisch verträgliche Salze von organischen Carbonsäuren, insbesondere der Zitronensäure oder der Milchsäure, oder
- eine oder mehrere Aminosäuren oder
- ein Mono- oder Disaccharid oder
- ein Zuckeralkohol
oder eine ihrer Mischungen zugesetzt ist.

13. Gewebekleber nach den Ansprüchen 1 bis 12, **dadurch gekennzeichnet, dass** eine im flüssigen oder gefrorenen Zustand stabile Thrombin-Zubereitung eingesetzt wird, die neben einem löslichen Calciumsalz und Natriumchlorid als Stabilisatoren
- eine Puffersubstanz
- einen Zucker oder Zuckeralkohol und/oder eine Aminosäure
und/oder
- ein Salz einer Mono- oder Polycarbonsäure oder
- ein Salz einer Mono- oder Polyhydroxycarbonsäure oder Mischungen der genannten Stabilisatoren enthalten kann.

14. Gewebekleber nach den Ansprüchen 1 bis 13, **dadurch gekennzeichnet, dass** die Thrombin-Zubereitung als Stabilisator einen nicht-kovalentbindenden Inhibitor enthält.

15. Gewebekleber nach den Ansprüchen 1 bis 14, **dadurch gekennzeichnet, dass** er eine Thrombin-Zubereitung enthält, welche durch eine hydrophobe Interaktionschromatographie und/oder eine Kationenaustauschchromatographie gereinigt wurde.

16. Gewebekleber nach den Ansprüchen 1 bis 15, **dadurch gekennzeichnet, dass** er oder seine Bestandteile einem oder mehreren Verfahren zur Inaktivierung oder Entfernung von Viren unterworfen wurden.

## Claims

1. Tissue adhesive containing
- a stabilized fibrinogen preparation storable in the liquid and/or frozen state, to which a chaotropic substance has been added, and
- a thrombin preparation
for use in the reduction or prevention of post-operative tissue adhesions.

2. Tissue adhesive according to Claim 1, **characterized in that** it additionally comprises a blood coagulation factor XIII-containing preparation which can also be mixed with the fibrinogen preparation.

3. Tissue adhesive according to Claims 1 and 2, **characterized in that** it contains an antifibrinolytic.

4. Tissue adhesive according to Claim 3, **characterized in that** it contains ε-aminocaproic acid as antifibrinolytic.

5. Tissue adhesive according to Claim 3, **characterized in that** it contains p-aminomethylbenzoic acid as antifibrinolytic.

6. Tissue adhesive according to Claim 3, **characterized in that** it contains aprotinin as antifibrinolytic.

7. Tissue adhesive according to Claims 1 to 6, **characterized in that** the plasminogen content of the fibrinogen component has been reduced by an immunoaffinity chromatography via coupled antibodies or an affinity chromatography via amino group-containing matrices.

8. Tissue adhesive according to Claims 1 to 7, **characterized in that** the fibrinogen component has a reduced plasminogen content, the ratio of plasminogen to fibrinogen being < 1.8 x 10⁻⁴ (w/w).

9. Tissue adhesive according to Claims 2 to 8, **characterized in that** the factor XIII-containing preparation has added to it
- a physiologically tolerable salt of an organic di-, tri- or tetracarboxylic acid, particularly of citric acid, and
- optionally further stabilizers and/or buffer substances for factor XIII.

10. Tissue adhesive according to Claim 9, **characterized in that** the factor XIII-containing preparation has added to it as further stabilizers
- a monosaccharide or disaccharide or a sugar alcohol and/or
- an amino acid from the group consisting of glycine, glycylglycine, alanine, cysteine, histidine, glutamine or a physiologically tolerable salt of glutamic acid or aspartic acid and/or
- a reducing or oxidation-preventing agent and/or
- a surface-active substance.

11. Tissue adhesive according to Claims 1 to 10, **characterized in that** the fibrinogen preparation has added to it one or more chaotropic substances from the group consisting of arginine, guanidine, citrulline, urea or the derivatives thereof or mixtures thereof.

12. Tissue adhesive according to Claims 1 to 11, **characterized in that** the fibrinogen preparation additionally has added to it as stabilizers
- an inorganic salt or
- one or more physiologically tolerable salts of organic carboxylic acids, more particularly of citric acid or lactic acid, or
- one or more amino acids or
- a monosaccharide or disaccharide or
- a sugar alcohol
or any mixture thereof.

13. Tissue adhesive according to Claims 1 to 12, **characterized in that** a thrombin preparation stable in the liquid or frozen state is used, which can contain in addition to a soluble calcium salt and sodium chloride as stabilizers
- a buffer substance
- a sugar or sugar alcohol and/or an amino acid
and/or
- a salt of a monocarboxylic acid or polycarboxylic acid or
- a salt of a monohydroxycarboxylic acid or polyhydroxycarboxylic acid or mixtures of said stabilizers.

14. Tissue adhesive according to Claims 1 to 13, **characterized in that** the thrombin preparation contains a non-covalent-binding inhibitor as stabilizer.

15. Tissue adhesive according to Claims 1 to 14, **characterized in that** it contains a thrombin preparation which was purified by means of a hydrophobic interaction chromatography and/or a cation-exchange chromatography.

16. Tissue adhesive according to Claims 1 to 15, **characterized in that** it or its constituents have been subjected to one or more methods for inactivating or removing viruses.

## Revendications

1. Colle pour tissus, contenant :
- une préparation de fibrinogène stabilisée, apte au stockage à l'état liquide et/ou congelé, à laquelle une substance chaotropique est ajoutée, et
- une préparation de thrombine,
pour une utilisation pour réduire ou empêcher les adhésions de tissus post-opératoires.

2. Colle pour tissus selon la revendication 1, **caractérisée en ce qu'**elle comprend en outre une préparation contenant le facteur de coagulation sanguine XIII, qui peut également être mélangée avec la préparation de fibrinogène.

3. Colle pour tissus selon les revendications 1 et 2, **caractérisée en ce qu'**elle contient un anti-fibrinolytique.

4. Colle pour tissus selon la revendication 3, **caractérisée en ce qu'**elle contient l'acide ε-aminocaproïque en tant qu'anti-fibrinolytique.

5. Colle pour tissus selon la revendication 3, **caractérisée en ce qu'**elle contient l'acide p-aminométhylbenzoïque en tant qu'anti-fibrinolytique.

6. Colle pour tissus selon la revendication 3, **caractérisée en ce qu'**elle contient l'aprotinine en tant qu'anti-fibrinolytique.

7. Colle pour tissus selon les revendications 1 à 6, **caractérisée en ce que** la teneur en plasminogène du composant fibrinogène a été réduite par chromatographie d'immuno-affinité par des anticorps couplés ou chromatographie d'affinité par des matrices contenant des groupes amino.

8. Colle pour tissus selon les revendications 1 à 7, **caractérisée en ce que** le composant fibrinogène présente une teneur en plasminogène réduite, le rapport entre le plasminogène et le fibrinogène étant < 1,8 x 10⁻⁴ (m/m).

9. Colle pour tissus selon les revendications 2 à 8, **caractérisée en ce que**
- un sel physiologiquement compatible d'un acide di-, tri- ou tétracarboxylique organique, notamment de l'acide citrique, et
- éventuellement d'autres stabilisateurs et/ou substances tampons pour le facteur XIII sont ajoutés à la préparation contenant le facteur XIII.

10. Colle pour tissus selon la revendication 9, **caractérisée en ce que**
- un mono- ou disaccharide ou un sucre-alcool et/ou
- un acide aminé du groupe constitué par la glycine, la glycylglycine, l'alanine, la cystéine, l'histidine, la glutamine ou un sel physiologiquement compatible de l'acide glutamique ou asparagique, et/ou
- un agent réducteur ou inhibant l'oxydation, et/ou
- une substance tensioactive,
sont ajoutés à la préparation contenant le facteur XIII en tant que stabilisateurs supplémentaires.

11. Colle pour tissus selon les revendications 1 à 10, **caractérisée en ce qu'**une ou plusieurs substances chaotropiques du groupe constitué par l'arginine, la guanidine, la citrulline, l'urée ou leurs dérivés ou leurs mélanges sont ajoutées à la préparation de fibrinogène.

12. Colle pour tissus selon les revendications 1 à 11, **caractérisée en ce que**
- un sel inorganique ou
- un ou plusieurs sels physiologiquement compatibles d'acides carboxyliques organiques, notamment de l'acide citrique ou de l'acide lactique, ou
- un ou plusieurs acides aminés, ou
- un mono- ou disaccharide, ou
- un sucre-alcool,
ou un de leurs mélanges, sont en outre ajoutés à la préparation de fibrinogène en tant que stabilisateurs.

13. Colle pour tissus selon les revendications 1 à 12, **caractérisée en ce qu'**une préparation de thrombine stable à l'état liquide ou congelé est utilisée, qui peut contenir en plus d'un sel de calcium soluble et de chlorure de sodium en tant que stabilisateurs
- une substance tampon,
- un sucre ou un sucre-alcool et/ou un acide aminé,
et/ou
- un sel d'un acide mono- ou polycarboxylique, ou
- un sel d'un acide mono- ou polyhydroxycarboxylique ou des mélanges des stabilisateurs mentionnés.

14. Colle pour tissus selon les revendications 1 à 13, **caractérisée en ce que** la préparation de thrombine contient en tant que stabilisateur un inhibiteur à liaison non covalente.

15. Colle pour tissus selon les revendications 1 à 14, **caractérisée en ce qu'**elle contient une préparation de thrombine qui a été purifiée par chromatographie d'interaction hydrophobe et/ou chromatographie d'échange de cations.

16. Colle pour tissus selon les revendications 1 à 15, **caractérisée en ce que** celle-ci ou ses constituants ont été soumis à un ou plusieurs procédés pour l'inactivation ou l'élimination de virus.
